# EUROPÄISCHE PATENTSCHRIFT

(11) **EP 1 021 131 B1**
(45) Veröffentlichungstag und Bekanntmachung des Hinweises auf die Patenterteilung: **05.01.2005**
(21) Anmeldenummer: 99940198.7
(22) Anmeldetag: 12.08.1999
(51) Int. Cl.: A61B 17/28

(54) **HANDGRIFF FÜR EIN MEDIZINISCHES INSTRUMENT**
HANDLE FOR MEDICAL INSTRUMENT
POIGNEE POUR UN INSTRUMENT MEDICAL

(30) Priorität: 12.08.1998 DE 19836481
(43) Veröffentlichungstag der Anmeldung: 26.07.2000
(73) Patentinhaber: Karl Storz GmbH & Co. KG, 78532 Tuttlingen (DE)
(72) Erfinder: BACHER, Uwe, D-78532 Tuttlingen (DE)
(74) Vertreter: Heuckeroth, Volker, Dr.
(86) Internationale Anmeldenummer: PCT/EP1999/005868
(87) Internationale Veröffentlichungsnummer: WO 2000/009021

(56) Entgegenhaltungen:
- EP-A- 0 640 319
- EP-A- 0 705 569
- US-A- 2 214 985
- US-A- 5 002 543
- US-A- 5 261 917
- US-A- 5 403 332
- US-A- 5 456 684
- US-A- 5 549 636

## Beschreibung

Die Erfindung betrifft einen Handgriff für ein medizinisches Instrument, das zwei unabhängig voneinander bewegliche Maulteile aufweist, mit einem Handgriffkörper, an dem zwei unabhängig voneinander bewegliche Griffteile zum wahlweisen Betätigen der Maulteile angeordnet sind, und mit einem Daumen-Griffteil, das einen Daumenring aufweist, wobei die beweglichen Griffteile distal von dem Daumen-Griffteil beabstandet am Handgriffkörper angeordnet sind und seitlich von diesem abstehen.

Ein derartiger Handgriff ist aus der US 5 261 917 A und aus der DE 694 03 583 T2, entsprechend der EP 0 640 319 B1, bekannt.

Ein eingangs genannter Handgriff ist für ein medizinisches Instrument vorgesehen, das zwei unabhängig voneinander bewegliche Maulteile aufweist. Derartige Zangen werden in der endoskopischen Chirurgie üblicherweise als Multifunktionsinstrumente eingesetzt, beispielsweise um Gewebe mit dem einen Maulteil zu schneiden und mit dem anderen Maulteil zur Entnahme des Gewebes aus dem Körper zu fassen. Dementsprechend ist das eine Maulteil als Schneidwerkzeug ausgebildet, während das andere Maulteil als Faßwerkzeug ausgestaltet ist. Beide Maulteile sind unabhängig voneinander beweglich, d.h. sie können unabhängig voneinander geschlossen und geöffnet werden. Es können auch beide Maulteile eine Schneidfunktion oder eine Faßfunktion besitzen.

Ein solcher Handgriff für ein medizinisches Instrument mit zwei unabhängig voneinander beweglichen Maulteilen und entsprechend zwei unabhängig voneinander beweglichen Griffteilen sollte so ausgebildet sein, daß der Arzt den Handgriff leicht bedienen kann, ohne die Bedienungsweise eines solchen Instruments neu lernen oder sich an eine neue Bedienungsweise gewöhnen zu müssen.

Ärzte sind bei einer überwiegenden Zahl von medizinischen Instrumenten an einen scherenartig bedienbaren Handgriff gewöhnt, wie er bspw. aus der DE-A-44 44 025 bekannt ist und von der Firma Karl Storz GmbH & Co., Tuttlingen, unter der Marke "Takeapart" vertrieben wird. Der Vorteil dieser Handgriffe besteht darin, daß diese mit Daumen und Zeige- und/oder Mittelfinger in der Art einer Schere gehalten und bedient werden können. Die beiden Griffteile sind dabei so ausgebildet, daß sie mit einer Handhaltung, in der der Handrücken im wesentlichen vertikal steht, gehalten werden können, was den Vorteil hat, daß das Handgelenk dabei gerade ist. Solche scherenartig zu bedienenden Handgriffe haben sich bei medizinischen Instrumenten als besonders ergonomisch erwiesen, weil mit Instrumenten, die scherenartig zu bedienende Handgriffe aufweisen, ermüdungsfrei gearbeitet werden kann.

Die zuvor genannten scherenartig ausgebildeten Handgriffe sind jedoch bislang nur für solche medizinischen Instrumente vorgesehen, die entweder nur ein bewegliches Maulteil oder zwei gemeinsam bewegliche Maulteile aufweisen. Dementsprechend weisen diese bekannten Handgriffe nur ein bewegliches Griffteil und ein unbewegliches Griffteil auf.

Aus der bereits genannten DE 694 03 533 T2 ist ein in der genannten Schrift als manipulierbare chirurgische Hand bezeichneter Handgriff bekannt, der drei bewegliche Griffteile aufweist, um Werkzeuge am distalen Ende eines Schafts unabhängig voneinander betätigen zu können. Von den drei beweglichen Griffteilen bildet eines das Daumen-Griffteil, und die beiden anderen bilden mit Zeige- und Mittelfinger bedienbare Griffteile. Die mit den Fingern betätigbaren Griffteile sind distal von dem Daumen-Griffteil und seitlich am Handgriffkörper angeordnet. Mit dieser Griffteilanordnung sollen die natürlichen Beweglichkeiten der menschlichen Hand auf die Werkzeuge am distalen Ende naturgetreu übertragen werden können. Dieser Handgriff weist an seinem proximalen Ende eine Abstützplatte auf, gegen die die Handfläche der dem Handgriff haltenden Hand anliegt, wobei der Handgriff einen "mechanischen Handschuh" darstellen soll, in dem der Daumen und zwei Finger des menschlichen Bedieners eingesteckt werden können. Dieser Handgriff wird dementsprechend stets gleichzeitig mit Daumen, Zeige- und Mittelfinger bedient, wobei jedem dieser Finger ein Griffelement am Handgriffkörper zugeordnet ist. Die Griffteile sind dabei in Form eines Dreiecks angeordnet. Abgesehen davon, daß dieser Handgriff aufgrund seiner zusätzlich vorgesehenen Funktionen sehr kompliziert aufgebaut ist, entspricht die Ausgestaltung dieses Handgriffs nicht derjenigen einer scherengriffartigen Anordnung. Vielmehr sind der Daumen, Zeige- und Mittelfinger beim Eingreifen in die jeweils vorgesehenen Fingerringe nach vorn von der Hand abgespreizt. Die Handhabung dieses Handgriffs erfordert eine längere Übung und besondere Geschicklichkeit.

Bei medizinischen Instrumenten mit zwei unabhängig voneinander beweglichen Maulteilen ist es mitunter erforderlich, das Instrument insgesamt um die Instrumentenachse zu drehen, um, nachdem bspw. mit dem einen Maulteil Gewebe abgetrennt wurde, das andere Maulteil an das abgetrennte Gewebe zu bringen, um dieses dann mit diesem Maulteil zu fassen. Dabei muß das gesamte Instrument je nach Lageanordnung der Maulteile einschließlich des Handgriffes um bis zu 180° gedreht werden.

Dies ist bei dem zuvor genannten bekannten Handgriff nur erschwert möglich, da das Daumen-Griffteil keine Drehung um die Längsachse des Handgriffs zuläßt.

Aus der US 5,403,332 ist ferner ein Handgriff für ein medizinisches Instrument mit zwei unabhängig voneinander beweglichen Maulteilen und entsprechend zwei unabhängig voneinander beweglichen Griffteilen bekannt. Dieser Handgriff ist in der Form eines T am proximalen Ende des Instrumentenschafts angeordnet. Die zwei beweglichen Griffteile stehen seitlich vom Instrumentenschaft ab und befinden sich distal vor dem proximalen feststehenden Haltegriff. Diese Handgriff wird so gehalten, daß der hintere querstehende Haltegriff in der Art eines Stabes in der Handinnenfläche liegt, so daß der Zeige- und Mittelfinger bzw. Ringfinger die als Tasten ausgebildeten beweglichen Griffteile umgreifen können, um diese zum Betätigen der Maulteile nach proximal gegen den querstehenden Haltegriff zu ziehen. Auch diese Ausgestaltung eines Handgriffs entspricht nicht derjenigen einer scherengriffartigen Griffanordnung.

Ferner ist aus der US 2,214,985 eine medizinische Zange mit zwei unabhängig voneinander beweglichen Maulteilen und zwei unabhängig voneinander beweglichen Griffteilen bekannt. Es ist ein drittes Griffteil distal vor und mittig zwischen den beweglichen Griffteilen angeordnet. Die beiden beweglichen Griffteile und das unbewegliche Griffteil weisen jeweils einen Fingerring zum Durchstecken eines Fingers bzw. Daumens auf. Das dritte unbewegliche Griffteil weist einen Fingerring auf, der in einer Ebene quer zur Längsachse der Zange angeordnet ist, wobei diese Ebene außerdem schräg zu den Ebenen der beiden Fingerringe der beweglichen Griffteile verläuft. Dieser Handgriff wird so gehalten, daß durch das distale unbewegliche Griffteil der Zeigefinger gesteckt wird, während der Daumen und der Mittelfinger durch die beiden proximalen seitlich angeordneten Fingerringe gesteckt werden. Es ergibt sich, daß diese Griffanordnung keine scherenartig bedienbare Griffanordnung darstellt, wie sie bei medizinischen Instrumenten mit nur einem beweglichen oder zwei zusammen beweglichen Maulteilen her bekannt sind, und an die der operierende Arzt gewöhnt ist.

Der Erfindung liegt daher die Aufgabe zugrunde, einen Handgriff der eingangs genannten Art für ein medizinisches Instrument, das zwei unabhängig voneinander bewegliche Maulteile aufweist, dahingehend weiterzubilden, daß der Handgriff ergonomisch ist, so daß ein ermüdungsfreies Arbeiten mit dem Instrument ermöglicht wird, und daß die Handhabung des Handgriffes zum Betätigen der beiden Maulteile vereinfacht ist.

Erfindungsgemäß wird diese Aufgabe hinsichtlich des eingangs genannten Handgriffes dadurch gelöst, daß das Daumen-Griffteil am proximalen Ende des Handgriffkörpers angeordnet ist, und daß das Daumen-Griffteil um eine Handgrifflängsachse drehbar ist, so daß das eine und das andere bewegliche Griffteil abwechselnd in eine mit dem Daumen-Griffteil eine scherenartig bedienbare Griffanordnung bildende Stellung bewegbar ist, in der der Daumenring und das mit diesem die scherenartig bedienbare Griffanordnung bildende Griffteil im wesentlichen in einer zur Handgrifflängsachse parallelen Ebene liegen.

Der erfindungsgemäße Handgriff ist demnach im Unterschied zu dem eingangs genannten bekannten Handgriff als doppelt scherenartige Griffanordnung ausgebildet, wobei jeweils eines der beweglichen Griffteile mit dem einen Daumen-Griffteil eine scherenartige Anordnung bilden kann. Dies hat den Vorteil, daß der Handgriff stets in der Art einer Schere gehalten werden kann, gleich ob das eine bewegliche Griffteil oder das andere betätigt werden soll, um das zugehörige Maulteil zu betätigen, wie es der Arzt bei Handgriffen für Instrumente mit einem beweglichen Maulteil oder mit zwei gemeinsam beweglichen Maulteilen gewohnt ist, die sich durch ihre Ergonomie bewährt haben und ein ermüdungsfreies Arbeiten ermöglichen. Somit muß sich der Arzt, der an einen scherenartigen Handgriff bei Instrumenten mit einem beweglichen Maulteil bereits gewöhnt ist, bei einem Instrument mit zwei unabhängig voneinander beweglichen Maulteilen nicht auf eine davon unterschiedliche Handhabungsweise umstellen. Unter scherenartiger Griffanordnung im Sinne der Erfindung wird verstanden, daß sich das Daumen-Griffteil und jeweils eines der beweglichen Griffteile in der Art einer Schere halten und betätigen lassen, wobei dies aber nicht zwangsläufig bedeutet, daß die beweglichen Griffteile verschwenkbar sind. Diese können auch als linear verschiebbare Griffteile ausgebildet sein.

Die von dem Daumenring und dem mit diesem die scherenartig bedienbare Griffanordnung bildende Griffteil aufgespannte gemeinsame Ebene enthält bevorzugt die Handgrifflängsachse.

Die seitliche Anordnung der beweglichen Griffteile am Handgriffkörper trägt zu einer verbesserten Ergonomie des Handgriffs bei, weil bei einer vertikalen Stellung der durch das Daumen-Griffteil und jeweils eines der beweglichen Griffteile gebildeten Scherengriffanordnung das bewegliche Griffteil unterhalb und vor dem Daumen-Griffteil liegt, was der anatomischen Lagebeziehung zwischen Daumen und Zeige- oder Mittelfinger bei vertikal gestelltem Handrücken entspricht.

Weiterhin ist es erfindungsgemäß vorgesehen, daß das Daumen-Griffteil, das einen Daumenring aufweist, drehbar ist. Dadurch wird der Vorteil erzielt, daß zum Drehen des Instruments, um nach Betätigung des einen Maulteils das andere Maulteil anschließend an die gleiche Stelle zu bringen, um dieses anschließend zu betätigen, der Daumen durch den Daumenring durchgesteckt bleiben kann. Somit muß der Handgriff zum Drehen des Instrumentes nicht vollständig losgelassen und nach Beendigung des Drehvorgangs erneut gegriffen werden, wodurch eine weitere wesentliche Vereinfachung der Handhabung des erfindungsgemäßen Handgriffes erreicht wird.

Der erfindungsgemäße Handgriff zeichnet sich somit durch seine hohe Ergonomie und seine leichte Handhabbarkeit aus.

Somit wird die der Erfindung zugrunde liegende Aufgabe vollkommen gelöst.

In einer bevorzugten Ausgestaltung sind die beiden beweglichen Griffteile umfänglich beabstandet angeordnet und bilden mit dem Daumenring eine im wesentlichen gleichschenklige Dreiecksanordnung.

Diese Maßnahme hat den Vorteil, daß die beiden beweglichen Griffteile am Handgriffkörper voneinander beabstandet sind, so daß das eine bewegliche Griffteil bei Betätigung des anderen Griffteiles nicht störend im Wege ist. Die Beabstandung wird maximal, wenn die beiden beweglichen Griffteile bevorzugterweise diametral gegenüberliegend angeordnet sind. Außerdem wird durch die Dreiecksanordnung der Vorteil erzielt, daß der Daumenring und die beiden beweglichen Griffteile zusammen eine symmetrische Griffanordnung bilden, so daß beide beweglichen Griffteile mit dem Daumen-Griffteil eine im wesentlichen identische scherenartige Griffanordnung bilden, so daß sich die Bedienungsweise des einen beweglichen Griffteiles von der Bedienung des anderen beweglichen Griffteiles im Zusammenwirken mit dem Daumenring nicht unterscheidet.

In einer weiteren bevorzugten Ausgestaltung sind der Daumenring und die beiden Griffteile gemeinsam in einer Ebene angeordnet.

Hierbei ist von Vorteil, daß die beiden beweglichen Griffteile somit umfänglich maximal, d.h. um 180°, voneinander beabstandet sind, so daß das "inaktive" Griffteil die Bedienung des "aktiven" Griffteils, das mit dem gerade zu betätigenden Maulteil in Wirkverbindung steht, nicht stört.

In einer weiteren bevorzugten Ausgestaltung steht der Daumenring seitlich von der Handgriffachse ab, derart, daß er mit dem zusammen mit ihm die scherengriffartige Griffanordnung bildenden Griffteil auf etwa einer Höhe bezüglich des Abstands von der Handgrifflängsachse liegt.

Diese Maßnahme ist besonders vorteilhaft, weil durch sie eine jeweilige Griffanordnung geschaffen wird, bei der der Daumen und der Zeige- und/oder Mittelfinger bei gerader Haltung des Handgelenks ihrer anatomischen Lage entsprechend auf gleicher Höhe liegen, ohne daß der Daumen abgespreizt werden muß.

In einer weiteren bevorzugten Ausgestaltung weisen die beweglichen Griffteile jeweils einen umfänglich geschlossenen oder teilumfänglich offenen Fingerring zum Durchstecken bzw. Anlegen eines Zeige- und/oder Mittelfingers auf.

Diese Maßnahme ist von Vorteil, wenn die beiden beweglichen Griffteile nicht gegen eine Federkraft betätigbar sind und somit nicht selbsttätig in ihre Ausgangsstellung zurückkehren, wie es bei Schneid- und Faßzangen üblicherweise vorgesehen ist, damit der Arzt zum Betätigen der Maulteile nicht gegen eine Federkraft arbeiten muß, so daß er die Schließkraft zum Schließen der Maulteile besser dosieren kann. In diesem Fall sind die vorgesehenen Fingerringe hinsichtlich der Handhabbarkeit der beweglichen Griffteile von Vorteil, da die Fingerringe ohne Umsetzen des Fingers nach distal und nach proximal verschwenkt werden können. Eine teilumfänglich offene Ausgestaltung des Fingerrings bspw. als Halb- oder Dreiviertelring hat den weiteren Vorteil, daß der betreffende Finger leichter in den Ring eingeführt werden kann.

In einer weiteren bevorzugten Ausgestaltung ist das Daumen-Griffteil in zumindest zwei Arbeitsstellungen, in denen der Daumenring mit jeweils einem der beweglichen Griffteile die scherengriffartig bedienbare Griffanordnung bildet, am Handgriffkörper drehfest arretiert.

Diese Maßnahme hat hinsichtlich des drehbar ausgestalteten Daumen-Griffteils den Vorteil, daß sich das Daumen-Griffteil während der Betätigung des jeweiligen Maulteils nicht unerwünscht verdrehen kann.

Dabei ist es weiterhin bevorzugt, wenn das Daumen-Griffteil beim Erreichen der beiden Drehstellungen mit dem Handgriffkörper selbsttätig verrastet.

Hierbei ist von Vorteil, daß die beiden die Arbeitsstellungen des Daumen-Griffteiles, wohldefiniert sind und daß durch die selbsttätige Verrastung des Daumen-Griffteils an dem Handgriffkörper keine weiteren Manipulationen, wie beispielsweise Betätigen eines Rastknopfes, erforderlich sind, um das Daumen-Griffteil drehfest zu arretieren.

In einer weiteren bevorzugten Ausgestaltung ist das Daumen-Griffteil in zumindest einer Drehrichtung über einen Vollkreis drehbar.

Diese Maßnahme hat den Vorteil, daß der Handgriffkörper mit den daran angeordneten beweglichen Griffteilen stets in die gleiche Drehrichtung gedreht werden kann, um das Daumen-Griffteil abwechselnd mit dem einen oder dem anderen beweglichen Griffteil in die die scherenartige Griffanordnung bildende Stellung zu bewegen. Bevorzugt ist es auch, wenn das Daumen-Griffteil in beiden Drehrichtungen über einen Vollkreis drehbar ist.

In einer weiteren bevorzugten Ausgestaltung ist das Daumen-Griffteil am Handgriffkörper relativ zu diesem axial bewegbar angebracht, wobei das Daumen-Griffteil in Richtung proximales Ende in eine axial nach proximal vom Handgriffkörper zurückgezogene Verdrehstellung bewegbar ist, in der es verdrehbar ist.

Diese Maßnahme hat den Vorteil, daß der Arzt das Daumen-Griffteil mit dem durch den Daumenring durchgesteckten Daumen aus der drehfesten Arbeitsstellung mit dem durch den Daumenring dirchgestecten Daumen nach proximal zurückziehen kann, wodurch das Daumen-Griffteil durch einfache Manipulation in seine Verdrehstellung bewegt werden kann. Dies ist insbesondere in Verbindung mit der selbsttätigen Verrastung des Daumen-Griffteils beim Erreichen der jeweiligen Arbeitsstellung von Vorteil, weil das Daumen-Griffteil durch Nachlassen der Zugkraft auf den Daumenring dann selbsstätig in die drehfeste Arbeitsstellung einrasten kann.

Dabei ist es weiterhin bevorzugt, wenn das Daumen-Griffteil und der Handgriffkörper mittels einer axial trennbaren Stift-Loch-Verbindung drehfest arretiert sind.

Durch diese Maßnahme wird auf konstruktiv vorteilhaft einfache Weise eine Verdrehsicherung für das Daumen-Griffteil in den Arbeitsstellungen geschaffen, die darüberhinaus eine leichte Entarretierung durch axiale Bewegung des Daumen-Griffteils ermöglicht.

In einer weiteren bevorzugten Ausgestaltung ist das Daumen-Griffteil mittels einer zwischen diesem und dem Handgriffkörper wirkenden Druckfeder nach distal vorgespannt.

Durch diese Maßnahme wird in Verbindung mit der zuvor genannten Stift-Sackloch-Verbindung ein konstruktiv vorteilhaft einfacher Verrastmechanismus geschaffen, der eine selbsttätige Verrastung des Daumen-Griffteils an dem Handgriffkörper ermöglicht, und der weiterhin zum Verdrehen des Daumen-Griffteils leicht zu lösen ist.

In einer weiteren bevorzugten Ausgestaltung sind die beweglichen Griffteile verschwenkbar am Handgriffkörper angeordnet.

Dies ist von Vorteil, weil schwenkbar gelagerte Griffteile als zweiarmige Hebel mit hoher Kraftübertragungskapazität ausgestaltet werden können.

In einer weiteren bevorzugten Ausgestaltung sind Arretiermittel vorgesehen, die in einer Arbeitsstellung des Daumen-Griffteils, in der das Daumen-Griffteil mit dem einen beweglichen Griffteil die scherenartig bedienbare Griffanordnung bildet, das mit dem anderen beweglichen Griffteil verbundene Maulteil in seiner Schließlage arretiert, und umgekehrt.

Diese Maßnahme hat den besonderen Vorteil, daß das "inaktive" Maulteil, das gerade nicht für einen Eingriff im Körper bewegt werden muß, lagefest in seiner Schließlage gehalten wird und sich nicht unerwünscht oder unbeabsichtigt öffnen und dabei ein Hindernis darstellen kann.

Dabei ist es weiterhin bevorzugt, wenn die beweglichen Griffteile jeweils über eine axial bewegliche Zugstange mit dem zugehörigen Maulteil in Wirkverbindung stehen, und die Arretiermittel die jeweilige Zugstange in einer Position unbeweglich arretieren, in der das zugehörige Maulteil geschlossen ist.

Hierbei ist von Vorteil, daß die Arretierung bzw. Sperre des "inaktiven" Maulteils durch Arretieren der Zugstange eine besonders wirksame Arretierung ermöglicht, die unmittelbar an der Zugstange selbst angreift.

In einer weiteren bevorzugten Ausgestaltung weisen die Arretiermittel eine drehbare Hülse auf, die am distalen Ende des Daumen-Griffteils angeordnet ist, die an einem Innenumfangsabschnitt einen nach innen ragenden Vorsprung aufweist, der in eine Ausnehmung in die jeweilige zu arretierende Zugstange eingreift, um diese Zugstange zu arretieren.

Durch diese Maßnahme wird eine konstruktiv besonders einfache Arretierungsmöglichkeit für das jeweils unbeteiligte Maulteil geschaffen, die vorteilhaft einfach in das Daumen-Griffteil integriert ist und in Verbindung mit der Drehbarkeit derselben eine leichte Bedienung ermöglicht.

Weiterhin ist es bevorzugt, wenn der Vorsprung mit der Ausnehmung durch Drehen des Daumen-Griffteils in die gewünschte Arbeitsstellung in Eingriff kommt.

In Verbindung mit der drehbaren Ausgestaltung des Daumen-Griffteils ist diese Ausgestaltung insbesondere hinsichtlich einer besonders einfachen Handhabung vorteilhaft, da die Arretierung des "inaktiven", d.h. unbeteiligten Maulteils mehr oder weniger automatisch erfolgt, wenn das Daumen-Griffteil um die Handgrifflängsachse gedreht wird, um mit dem anderen Griffteil die scherengriffartig bedienbare Griffanordnung zu bilden.

Die vorstehend genannten Vorteile des erfindungsgemäßen Handgriffs werden bei einem medizinischen Instrument genutzt, das zwei unabhängig voneinander bewegliche Maulteile aufweist.

Bei dem medizinischen Instrument ist es dabei weiterhin bevorzugt, wenn die Schließrichtung des jeweiligen Maulteils und die Bewegungsrichtung des entsprechenden beweglichen Griffteils bezüglich des Daumen-Griffteils in etwa der gleichen Ebene liegen.

Wenn die beiden Maulteile bezüglich der aus dem jeweiligen beweglichen Griffteil und dem Daumen-Griffteil gebildeten Griffanordnung so angeordnet sind, daß die Schließrichtung des jeweiligen Maulteils und die Bewegungsrichtung des entsprechenden beweglichen Griffteils bezüglich des Daumen-Griffteils in etwa der gleichen Ebene liegen, wird die Handhabung des Instruments weiter vereinfacht. Das mit dem dem erfindungsgemäßen Handgriff ausgestattete medizinische Instrument mit zwei unabhängig voneinander beweglichen Maulteilen läßt sich somit hinsichtlich beider Maulteile wie ein herkömmliches Instrument mit nur einem beweglichen Maulteil, das einen scherenartigen Griff aufweist, bedienen.

Insgesamt ermöglicht es die Erfindung, zwei herkömmliche vollständige Instrumente mit einem beweglichen Maulteil und einem Handgriff zu einem Instrument mit zwei unabhängig voneinander beweglichen Maulteilen zu kombinieren, das nur einen Handgriff aufweist, ohne daß die Ergonomie des Handgriffs des kombinierten Instruments im Vergleich zu den Handgriffen der herkömmlichen Einzelinstrumente verschlechtert ist.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf die Figuren näher beschrieben. Es zeigen:
- Fig. 1: ein mit einem erfindungsgemäßen Handgriff ausgestattetes medizinisches Instrument mit zwei unabhängig voneinander beweglichen Maulteilen;
- Fig. 2: eine Seitenansicht des Handgriffs in Fig. 1 in Alleinstellung, die die Stellung des Handgriffs im Gebrauch zeigt;
- Fig. 3: den Handgriff in Fig. 2 in einer perspektivischen Darstellung, die die Verdrehbarkeit der beiden beweglichen Griffteile relativ zu dem Daumen-Griffteil des Handgriffes veranschaulicht;
- Fig. 4: einen Längsschnitt entlang der Linie IV-IV in Fig. 1 durch einen Abschnitt des Handgriffs in Fig. 1;
- Fig. 5: ein weiteres Ausführungsbeispiel eines erfindungsgemäßen Handgriffs in einer perspektivischen Gesamtdarstellung;
- Fig. 6: ein Teil des Handgriffs in Fig. 5, wobei das Handgriffgehäuse weggelassen wurde; und
- Fig. 7: eine Seitenansicht des Handgriffs in Fig. 5 und 6 in einer schematischen aufgebrochenen Darstellung.

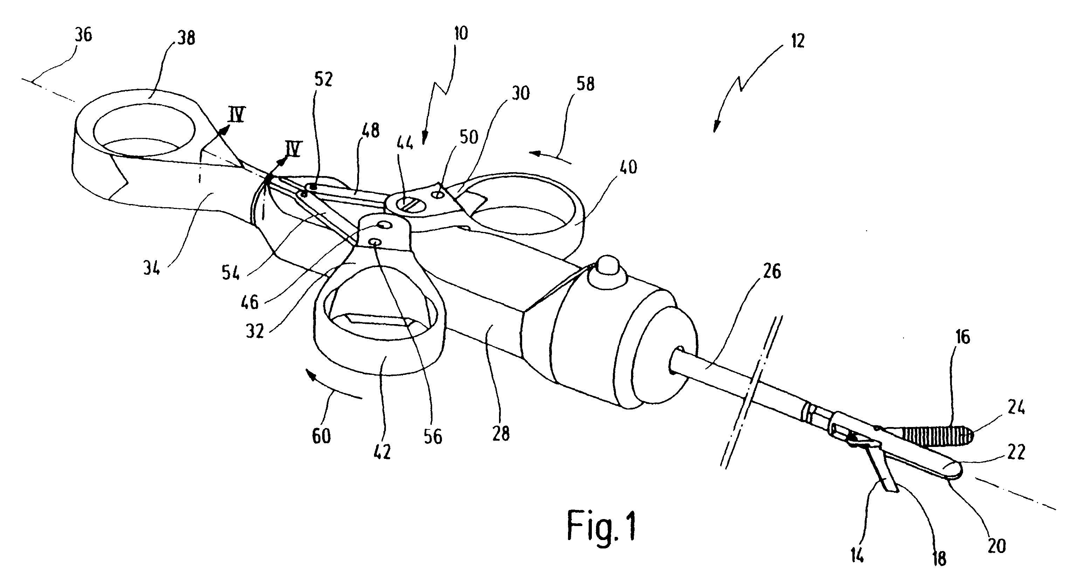

Fig. 1 zeigt einen mit dem allgemeinen Bezugszeichen 10 versehenen Handgriff für ein mit dem allgemeinen Bezugszeichen 12 versehenes medizinisches Instrument.

Das medizinische Instrument 12 weist an seinem distalen Ende ein erstes bewegliches Maulteil 14 und ein zweites bewegliches Maulteil 16 auf. Das erste Maulteil 14 und das zweite Maulteil 16 sind unabhängig voneinander beweglich, d.h. sie können unabhängig voneinander geöffnet bzw. geschlossen werden.

In dem dargestellten Ausführungsbeispiel hat das erste Maulteil 14 eine Schneidfunktion und ist dazu mit einer Schneide 18 ausgebildet, die beim Schließen des Maulteils 14 mit einer Schneide 20 eines unbeweglichen Maulbasisteils 22 schneidend zusammenwirkt. Mit dem ersten Maulteil 14 kann somit im menschlichen oder tierischen Körper Gewebe schneidend abgetrennt werden.

Das zweite Maulteil 16 ist als Faßwerkzeug ausgebildet und weist dazu eine gezahnte Fläche 24 auf, die mit einer an dem Maulbasisteil 22 ausgebildeten, in Fig. 1 nicht sichtbaren entsprechend gezahnten Fläche zum Fassen von Gewebe zusammenwirkt.

Das Instrument 12 wird bei einem endoskopischen chirurgischen Eingriff somit zum Schneiden von Gewebe sowie zum Fassen des abgeschnittenen Gewebes verwendet, um das abgetrennte Gewebe aus dem Körper zu entfernen. Es versteht sich jedoch, daß auch andere Maulteile mit anderen Funktionen im Rahmen der Erfindung verwendbar sind.

Der Handgriff 10 ist am proximalen, d.h. am maulteilfernen, Ende des Instrumentes 12 angeordnet, wobei sich ein Schaft 26 von dem Handgriff 10 bis etwa zu den Maulteilen 14 und 16 erstreckt.

Der Handgriff 10 weist einen Handgriffkörper 28 in Form eines Gehäuses auf. An dem Handgriffkörper 28 sind ein erstes bewegliches Griffteil 30 und ein zweites bewegliches Griffteil 32 angeordnet.

Am proximalen Ende des Handgriffkörpers 28 ist ein Daumen-Griffteil 34 angeordnet, das, wie noch näher beschrieben wird, drehbar ist.

Das erste bewegliche Griffteil 30 und das zweite bewegliche Griffteil 32 sind distal von dem Daumen-Griffteil 34 angeordnet und stehen seitlich von dem Handgriffkörper 28 ab.

Das erste bewegliche Griffteil 30 und das zweite bewegliche Griffteil 32 sind bezüglich einer Handgrifflängsachse 36, die die Längsmittelachse des Handgriffs 10 und auch des gesamten Instruments 12 bildet, symmetrisch zueinander an dem Handgriffkörper 28 einander gegenüberliegend angeordnet.

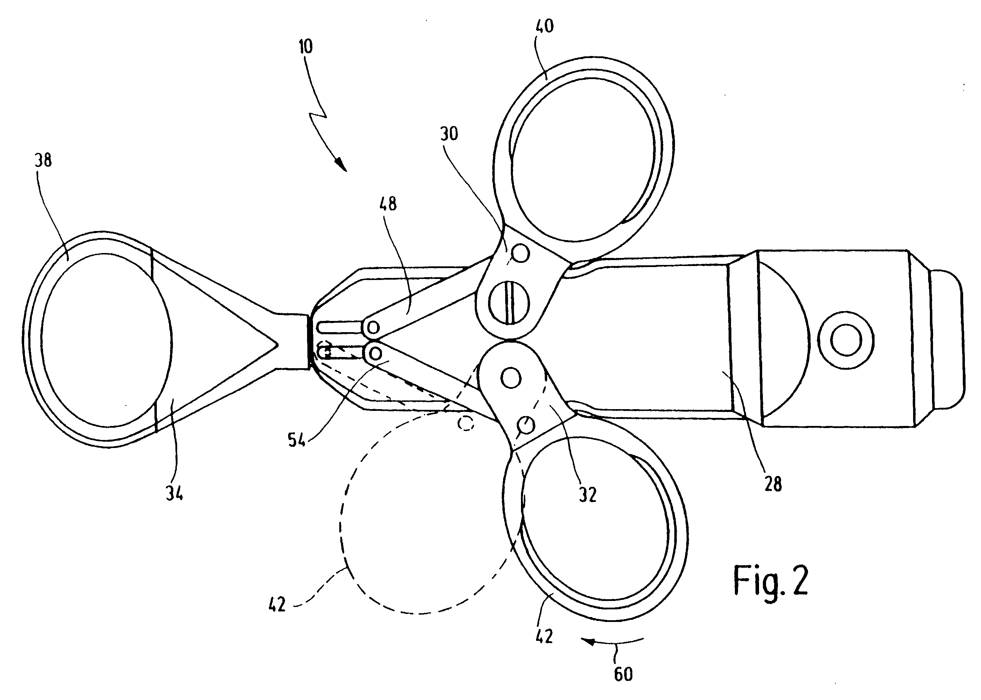

Das Daumen-Griffteil 34 und die beweglichen Griffteile 30 und 32 liegen dabei im wesentlichen in einer gemeinsamen Ebene und spannen in ihrer Stellung gemäß Fig. 1 und 2 dabei ein etwa gleichschenkliges Dreieck auf.

Das Daumen-Griffteil 34 weist einen Daumenring 38 zum Durchstecken eines Daumens einer Hand auf, während das erste bewegliche Griffteil 30 einen Fingerring 40 und das zweite bewegliche Griffteil 32 einen Fingerring 42 zum Durchstecken eines Fingers derselben Hand aufweist, wie hiernach noch anhand der Beschreibung der Handhabung des Handgriffes 10 näher beschrieben wird.

Das erste bewegliche Griffteil 30 dient der Betätigung des Öffnens und Schließens des ersten Maulteils 14, während das zweite bewegliche Griffteil 32 der Betätigung des Öffnens und Schließens des zweiten Maulteils 16 dient. Das erste Griffteil 30 und das zweite Griffteil 32 sind unabhängig voneinander beweglich an dem Handgriffkörper 28 angebracht.

Dazu ist das erste bewegliche Griffteil 30 über ein Scharniergelenk 44 und das zweite beweglich Griffteil 32 über ein Scharniergelenk 46 um die jeweilige Scharnierachse verschwenkbar an dem Handgriffkörper 28 befestigt.

Die Kraftübertragung von dem ersten beweglichen Griffteil 30 auf das erste bewegliche Maulteil 14 erfolgt über einen Betätigungsmechanismus, der ein Hebelgestänge 48 aufweist. Dieses ist mit seinem distalen Ende an einer Anlenkstelle 50, die von dem Scharniergelenk 44 beabstandet ist, an dem beweglichen Griffteil 30 angelenkt. Mit seinem proximalen Ende 52 steht das Hebelgestänge mit einer sich durch den Handgriffkörper 28 und den Schaft 26 bis zu dem ersten beweglichen Maulteil 14 erstreckenden in Fig. 1 nicht sichtbaren ersten Zugstange in Verbindung, die mit letzterem kraftschlüssig verbunden ist.

In gleicher Weise ist das zweite bewegliche Griffteil 32 über ein Hebelgestänge 54, dessen distales Ende an einem Anlenkungspunkt 56 an dem beweglichen Griffteil 32 angelenkt ist, und über eine mit dem Hebelgestänge 54 in Verbindung stehende, ebenfalls nicht dargestellte zweite Zugstange mit dem zweiten beweglichen Maulteil 16 kraftschlüssig verbunden.

In Fig. 1 sind das erste bewegliche Griffteil 30 und das zweite bewegliche Griffteil 32 in ihrer jeweils maximal distalen Schwenkstellung dargestellt, in der das erste Maulteil 14 und das zweite Maulteil 16 ihre maximale Offenstellung einnehmen. Wird nun bspw. das erste bewegliche Griffteil 30 in Richtung eines Pfeiles 58 zum proximalen Ende hin verschwenkt, wird das erste bewegliche Maulteil 14 geschlossen. Zum Öffnen des ersten beweglichen Maulteiles 14 aus der Schließlage wird das erste bewegliche Griffteil 30 entgegen dem Pfeil 58 verschwenkt.

Unabhängig von dem ersten beweglichen Griffteil 30 läßt sich das zweite bewegliche Griffteil 32 gemäß einem Pfeil 60 zum proximalen Ende hin verschwenken, wodurch das zweite bewegliche Maulteil 16 geschlossen und bei umgekehrter Bewegung wieder geöffnet wird.

Für einen Einsatz des Instrumentes 12 bei einer Operation wird der Handgriff 10 in einer Lage gehalten, wie sie in Fig. 2 dargestellt ist. In dieser Stellung des Handgriffs 10 steht die von dem beweglichen Griffteil 32 und dem Daumen-Griffteil 34 gebildete Scherengriffanordnung etwa vertikal.

In dieser Stellung des Handgriffs 10 läßt sich das Instrument 12 mit geradem Handgelenk halten, indem ein Daumen einer Hand durch den Daumenring 38 und ein Finger derselben Hand, beispielsweise der Zeige- oder der Mittelfinger oder beide, durch den Fingerring 42 des zweiten beweglichen Griffteils 32 durchgesteckt werden. Der Fingerring 42 des zweiten beweglichen Griffteils 32 ist in der in Fig. 2 dargestellten Stellung des Handgriffs 10 unterhalb des Daumenrings 38 angeordnet, was der anatomischen Lagebeziehung zwischen Daumen und Zeigefinger entspricht, wenn die Hand mit etwa vertikal stehendem Handrücken gehalten wird.

Mittels des durch den Fingerring 42 durchgesteckten Fingers und des durch den Daumenring 38 durchgesteckten Daumen läßt sich nun das zweite bewegliche Griffteil 32 zum Schließen und Öffnen des zweiten beweglichen Maulteils 16 in der Art einer Schere betätigen.

Aufgrund der symmetrischen Anordnung der beweglichen Griffteile 30 und 32 kann nun der Handgriff 10 um die Handgrifflängsachse 36 um 180° gedreht werden, so daß dann das erste bewegliche Griffteil 30 bezüglich der Vertikalen unterhalb des Daumen-Griffteils 34 liegt, während dann das zweite bewegliche Griffteil 32 über dem Daumen-Griffteil 34 zu liegen kommt. Nach einer Drehung des Handgriffs 10 um 180° bilden dann das Daumen-Griffteil 34 und das erste bewegliche Griffteil 30 eine scherenartig bedienbare Anordnung, so daß auch das erste Maulteil 14 mit der gleichen Handhaltung wie zuvor für das zweite bewegliche Maulteil 16 beschrieben in der Art einer Schere betätigt werden kann.

Um ausgehend von Fg. 2 das erste Griffteil 30 mt dem Daumen-Griffteil 34 in eine eine scherenartig bedienbare Griffanordnung bildende Stellung zu bewegen, ist es jedoch nicht erforderlich, den gesamten Handgriff 10 zu drehen, da das Daumen-Griffteil 34 an dem Handgriffkörper 28 um die Handgrifflängsachse 36 drehbar ist.

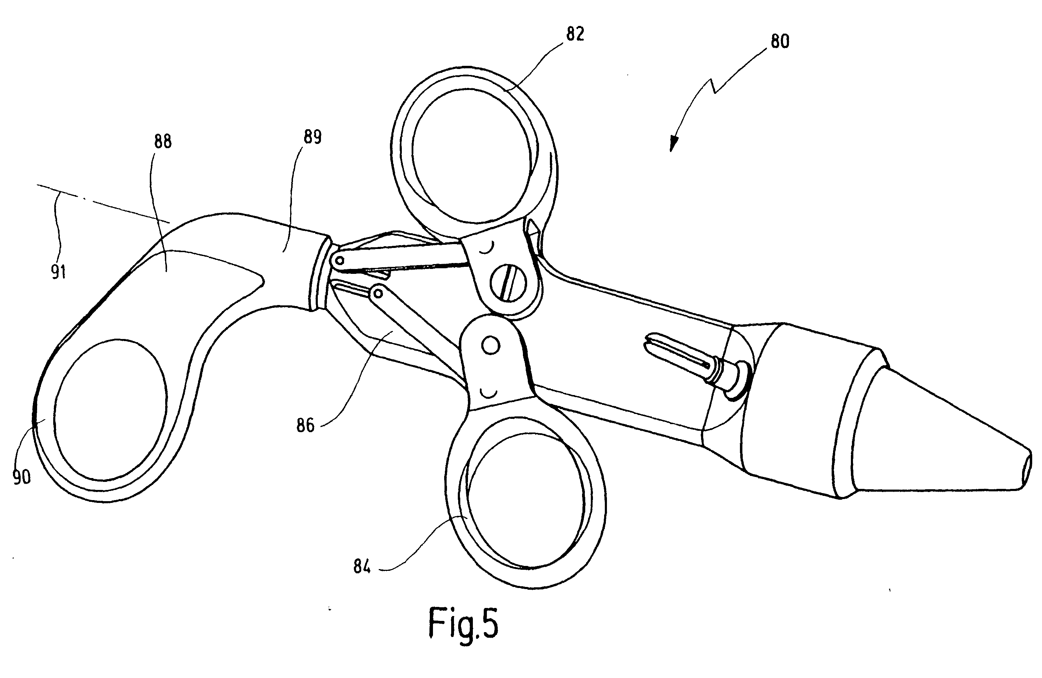

Dazu weist das Daumen-Griffteil 34 einen hülsenartigen Fortsatz 62 auf (vgl. Fig. 4), der einen zylindrischen Endabschnitt 64 des Handgriffkörpers 28 übergreift. Der hülsenartige Fortsatz 62 ist auf dem zylindrischen Endabschnitt 64 axial in Richtung eines Pfeils 66 verschiebbar, wobei eine Druckfeder 68, die einerseits an dem Daumen-Griffteil 34 und andererseits an dem Handgriffkörper 28 befestigt ist, das Daumen-Griffteil 34 entgegen dem Pfeil 66 nach distal spannt.

An dem zylindrischen Endabschnitt 64 des Handgriffkörpers 28 ist ferner nichtmittig ein Stift 70 befestigt, der aus dem proximalen Ende des Abschnitts 64 hervorragt und in ein in dem Daumen-Griffteil 34 ausgespartes Sackloch oder Loch 72 eingreift. Dem Loch 72 diametral gegenüberliegend ist ein weiteres, nicht dargestelltes entsprechendes Loch in dem Daumen-Griffteil 34 vorgesehen. Wenn der Stift 70 in das Loch 72 oder das entsprechend vorgesehene zweite Loch eingreift, ist das Daumen-Griffteil 34 an dem Handgriffkörper 28 drehfest arretiert. Das Daumen-Griffteil 34 weist somit zwei Arbeitsstellungen bezüglich der beweglichen Griffteile 30 und 32 auf, in denen es drehfest bezüglich dieser beweglichen Griffteile 30 und 32 ist. In diesen Arbeitsstellungen bilden das Daumen-Griffteil 34 und das bewegliche Griffteil 30 bzw. das bewegliche Griffteil 32 jeweils die Scherengriffanordnung.

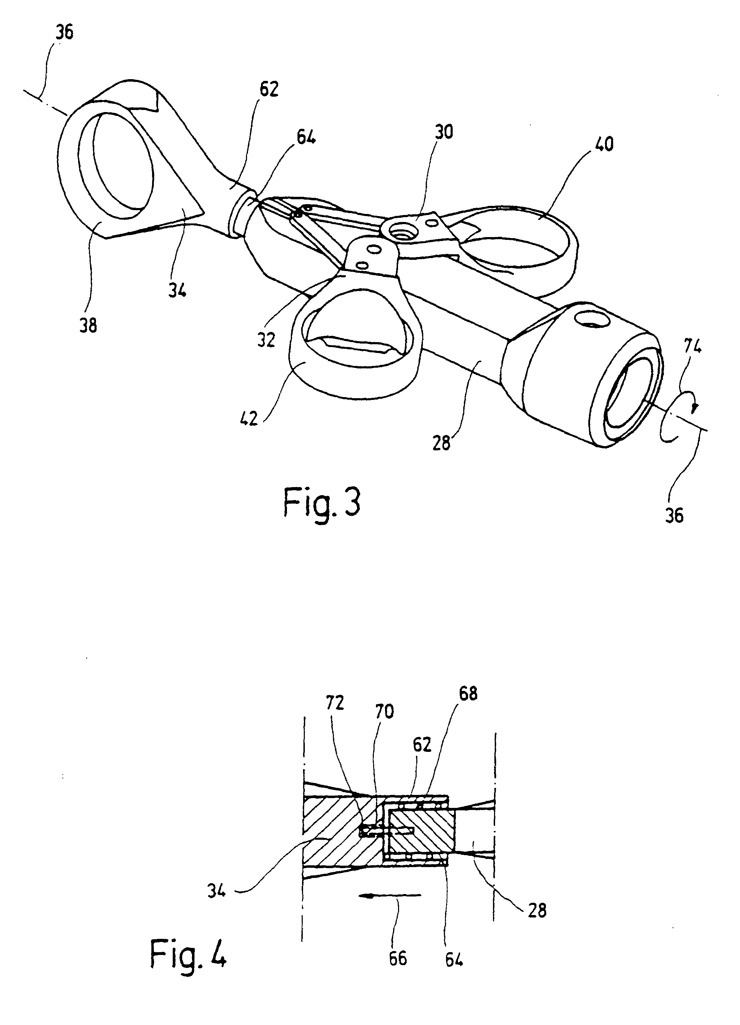

Um das Daumen-Griffteil 34 aus der in Fig 2 gezeigten Arbeitsstellung zu entarretieren, wird mittels des durch den Daumenring 38 durchgesteckten Daumens das Daumen-Griffteil 34 in Richtung des Pfeiles 66 gemäß Fig. 4 nach proximal gezogen, bis der Stift 70 aus dem Loch 72 herausgetreten ist. Nun läßt sich das Daumen-Griffteil 34 an dem Handgriffkörper 28 verdrehen, wie in Fig. 3 dargestellt ist. Dazu muß das Instrument nicht aus dem menschlichen oder tierischen Körper entfernt werden, sondern kann in situ verbleiben. Während der Daumenring 38 mittels des durchgesteckten Daumens festgehalten wird, wird der Handgriffkörper 28 um die Handgrifflängsachse 36 gemäß einem Pfeil 74 um etwa 180° gedreht, wodurch auch die beiden Maulteile 14 und 16 um die Handgrifflängsachse 36 mitgedreht werden. Während des Drehens muß der Daumenring 38 nicht zurückgezogen gehalten werden, da sich nun der Stift 70 des Endabschnittes 64 gegen die proximale Wand des hülsenartigen Fortsatzes 62 abstützen kann und dadurch das Daumen-Griffteil zurückgezogen hält. Sobald die um 180° versetzte Drehstellung erreicht ist, greift der Stift 70 selbsttätig unter der Wirkung der Druckfeder 68 in das dem Loch 72 diametral gegenüberliegend angeordnete Loch ein, wodurch das Daumen-Griffteil 34 wieder an dem Handgriffkörper 28 drehfest verrastet ist. Nun kann derselbe Finger, der zuvor zum Betätigen des zweiten beweglichen Griffteils 32 verwendet wurde, durch den Fingerring 40 des ersten beweglichen Griffteils 30 durchgesteckt werden, um das erste bewegliche Griffteil 30 und damit das erste bewegliche Maulteil 14 zu betätigen.

Ist es gewünscht, daß anschließend wiederum das zweite Maulteil 16 betätigt werden soll, kann der zuvor beschriebene Vorgang des Verdrehens der beweglichen Griffteile 30 und 32 relativ zu dem Daumen-Griffteil 34 wiederholt werden, wobei die Verdrehung bezüglich des Daumen-Griffteils 34 stets in der gleichen Drehrichtung erfolgen kann, da das Daumen-Griffteil 34 über einen Vollkreis an dem Handgriffkörper 28 drehbar angebracht ist.

Die Maulteile 14 und 16 sind bezüglich der beweglichen Griffteile 30 und 32 und dem Daumen-Griffteil 34 derart angeordnet, daß die Schließrichtung des Maulteils 16 und die Bewegungsrichtung des beweglichen Griffteils 32 bezüglich des Daumen-Griffteils 34 in etwa der gleichen Ebene liegen. Entsprechendes gilt für das bewegliche Maulteil 14. Wie aus Fig. 1 weiter hervorgeht, sind das erste beweglich Maulteil 14 und das dazugehörige bewegliche Griffteil 30 sowie das beweglich Maulteil 16 und das dazugehörige bewegliche Griffteil 32 bezüglich der Handgrifflängsachse 36 gegenüberliegend angeordnet.

In Fig. 5 bis 7 ist ein weiteres Ausführungsbeispiel eines mit dem allgemeinen Bezugszeichen 80 versehenen Handgriffs dargestellt, von dem im folgenden die Unterschiede zu dem Handgriff 10 gemäß Fig. 1 bis 4 beschrieben werden. Nicht beschriebene Merkmale des Handgriffs 80 sind mit den entsprechenden Merkmalen des Handgriffs 10 identisch oder vergleichbar.

Der Handgriff 80 kann bspw. anstelle des Handgriffs 10 bei dem medizinischen Instrument 10 gemäß Fig. 1 verwendet werden.

Der Handgriff 80 weist ein erstes bewegliches Griffteil 82 sowie ein zweites bewegliches Griffteil 84 auf, wobei das erste bewegliche Griffteil 82 und das zweite bewegliche Griffteil 84 wieder unabhängig voneinander beweglich sind, um bspw. die beiden Maulteile 14 und 16 am distalen Ende des medizinischen Instruments 12 unabhängig voneinander zu bewegen.

Am proximalen Ende eines Handgriffkörpers 86 ist ein Daumen-Griffteil 88 angeordnet. Das Daumengriffteil 88 weist einen Daumenring 90 auf.

Das Daumen-Griffteil 88 ist um eine Handgrifflängsachse 91 des Handgriffkörpers 86 drehbar, und zwar in beiden Drehrichtungen um zumindest 180°.

Das Daumen-Griffteil 88 ist mit seinem Befestigungsabschnitt 89 konzentrisch zur Handgrifflängsachse 91 am Handgriffkörper 86 befestigt, wobei der Daumenring 90 seitlich von der Handgrifflängsachse 91 absteht.

In der in Fig. 5 bis 7 gezeigten Arbeitsstellung bildet das Daumen-Griffteil 88 mit dem beweglichen Griffteil 84 eine scherenartig bedienbare Griffanordnung.

Durch Drehen des Daumengriffteils 88 um 180° um die Handgrifflängsachse 91 bildet das Daumengriffteil 88 dann mit dem beweglichen Griffteil 82 eine scherenartig bedienbare Griffanordnung.

Wie aus Fig. 5 bis 7 hervorgeht, liegt der Daumenring 90 zusammen mit dem beweglichen Griffteil 84, genauer gesagt dessen Fingerring, auf etwa der gleichen Höhe bezüglich des Abstands von der Handgrifflängsachse 91, wodurch eine besonders ergonomische Handhaltung und Bedienung des beweglichen Griffteils 84 zum Betätigen des nicht dargestellten zugehörigen Maulteils am distalen Ende des Instruments geschaffen wird. Durch Drehen des Daumen-Griffteils 88 um 180° um die Handgrifflängsachse 91 wird die gleiche ergonomisch günstige Anordnung mit dem anderen beweglichen Griffteil 82 geschaffen.

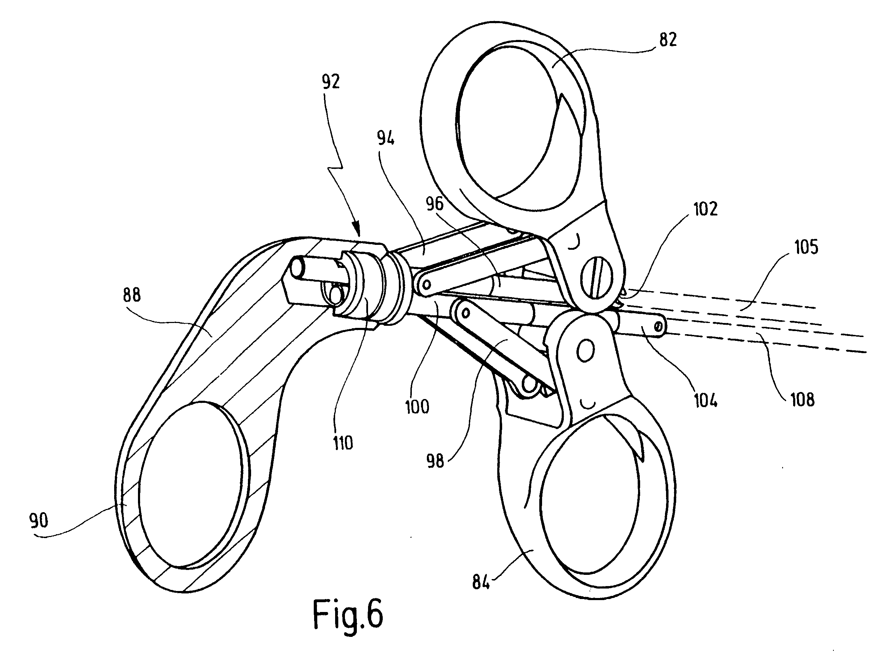

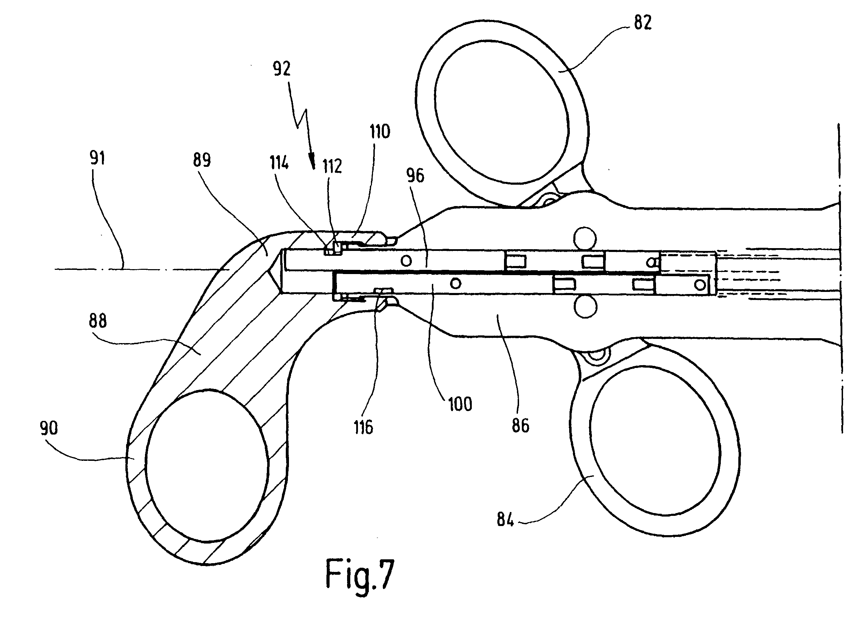

Anhand von Fig. 6 und 7 werden nun Arretiermittel 92, mit denen wahlweise das erste bewegliche Griffteil 82 oder das zweite bewegliche Griffteil 84 unbeweglich arretiert werden können, näher beschrieben.

Das erste bewegliche Griffteil 82 steht über ein Gestänge 94 mit einer ersten Zugstange 96 kraftschlüssig in Verbindung, um das zugehörige Maulteil am distalen Ende des Instruments öffnen und schließen zu können, wie dies ebenfalls bei dem Handgriff 10 gemäß Fig. 1 bis 4 vorgesehen ist.

Das zweite bewegliche Griffteil 84 steht entsprechend über ein Gestänge 98 mit einer zweiten Zugstange 100 kraftschlüssig in Verbindung, um das andere Maulteil entsprechend zu öffnen oder zu schließen. Die Zugstangen 96 und 100 sind dazu unabhängig voneinander axial beweglich.

Die erste Zugstange 96 und die zweite Zugstange 100 weisen jeweils Aufhängungen 102 bzw. 104 auf, so daß mit den Maulteilen verbundene, mit unterbrochenen Linien angedeutete, mit den Maulteilen verbundene Zugstangenabschnitte 106 und 108 vom Handgriff 80 getrennt werden können.

Das Daumen-Griffteil 88 weist eine Hülse 110 auf, in die die Zugstangen 96 und 100 eingreifen.

Die Hülse 110 weist gemäß Fig. 7 an einem Innenumfangsabschnitt einen nach innen ragenden Vorsprung 112 auf.

Die Zugstange 96 weist an ihrem proximalen Ende eine Ausnehmung 114 auf, während die Zugstange 100 ebenfalls eine Ausnehmung 116 aufweist, die der Ausnehmung 114 der Zugstange 96 diametral gegenüberliegend angeordnet ist. Die Zugstangen 96 und 100 sind unverdrehbar in dem Handgriffkörper 86 angeordnet.

In Fig. 6 und 7 ist die Zugstange 96 in ihre axial maximal proximale Stellung zurückgezogen, in der auch das erste bewegliche Griffteil 82 nach proximal zurückgeschwenkt ist.

In dieser Stellung des beweglichen Griffteils 82 bzw. in dieser Stellung der Zugstange 96 befindet sich das zugehörige Maulteil am distalen Ende des Instruments in seiner Schließstellung.

Der Vorsprung 112 in der Hülse 110 des Daumen-Griffteils 88 greift in dieser Stellung in die Ausnehmung 114 der Zugstange 96 ein, wodurch die Zugstange 96 und damit das bewegliche Griffteil 82 unbeweglich arretiert sind. Somit ist auch das zugehörige Maulteil in seiner Schließstellung arretiert.

Das zweite bewegliche Griffteil 84, das in dieser Drehstellung des Daumen-Griffteils 88 mit diesem die scherenartig bedienbare Griffanordnung bildet, ist dagegen frei schwenkbar, so daß das mit diesem beweglichen Griffteil 84 in Wirkverbindung stehende Maulteil durch Betätigen des Griffteils 84 geöffnet und geschlossen werden kann.

Durch Drehen des Daumengriffteils 88 um die Handgrifflängsachse 92 um 180° gibt der Vorsprung 112 die Ausnehmung 114 in der Zugstange 96 frei, und kommt dann mit der Ausnehmung 116 der Zugstange 100 in Eingriff, so daß die Zugstange 100 und damit das bewegliche Griffteil 84 und das zugehörige Maulteil unbeweglich in der Schließstellung arretiert sind. Das bewegliche Griffteil 84 wird dazu zuvor in Richtung proximales Ende verschwenkt, um die Zugstange 100 in die maximal proximale Stellung zu bringen, damit der Vorsprung 112 in die Ausnehmung 116 eingreifen kann.

Durch die Ausgestaltung der Arretiermittel 92 ist somit stets gewährleistet, daß dasjenige bewegliche Griffteil, daß mit dem Daumen-Griffteil 88 nicht die scherenartig bedienbare Griffanordnung bildet, arretiert bzw. inaktiviert.

Auch das Daumen-Griffteil 88 ist in den beiden Arbeitsstellungen dreharretiert.

## Patentansprüche

1. Handgriff für ein medizinisches Instrument (12), das zwei unabhängig voneinander bewegliche Maulteile (14, 16) aufweist, mit einem Handgriffkörper (28; 86), an dem zwei unabhängig voneinander bewegliche Griffteile (30, 32; 82, 84) zum wahlweisen Betätigen der Maulteile (14, 16) angeordnet sind, und mit einem Daumen-Griffteil (34; 88), das einen Daumenring (38; 90) aufweist, wobei die beweglichen Griffteile (30, 32; 82, 84) distal von dem Daumen-Griffteil (34; 88) beabstandet am Handgriffkörper (28; 86) angeordnet sind und seitlich von diesem abstehen, **dadurch gekennzeichnet, daß** das Daumen-Griffteil (34; 88) am proximalen Ende des Handgriffkörpers (28; 86) angeordnet ist, und daß das Daumengriffteil (34; 88) um eine Handgrifflängsachse (36; 91) drehbar ist, so daß das eine und das andere bewegliche Griffteil (30, 32; 82, 84) abwechselnd in eine mit dem Daumen-Griffteil (34; 88) jeweils eine scherenartig bedienbare Griffanordnung bildende Stellung bewegbar ist, in der der Daumenring (38; 90) und das mit diesem die scherenartig bedienbare Griffanordnung bildende Griffteil (30, 32; 82, 84) im wesentlichen in einer zur Handgrifflängsachse (36; 91) parellelen Ebene liegen.

2. Handgriff nach Anspruch 1, **dadurch gekennzeichnet, daß** die beiden beweglichen Griffteile (30, 32; 82, 84) umfänglich beabstandet angeordnet sind und mit dem Daumenring (38; 90) eine im wesentlichen gleichschenklige Dreiecksanordnung bilden.

3. Handgriff nach Anspruch 1 oder 2, **dadurch gekennzeichnet, daß** der Daumenring (38; 90) und die beiden Griffteile (30, 32; 82, 84) gemeinsam in einer Ebene angeordnet sind.

4. Handgriff nach einem der Ansprüche 1 bis 3, **dadurch gekennzeichnet, daß** der Daumenring (90) seitlich von der Handgriffachse (91) absteht, derart, daß er mit dem zusammen mit ihm die scherengriffartige Griffanordnung bildenden Griffteil (82, 84) auf etwa einer Höhe bezüglich des Abstands von der Handgrifflängsachse (91) liegt.

5. Handgriff nach einem der Ansprüche 1 bis 4, **dadurch gekennzeichnet, daß** die beweglichen Griffteile (30, 32; 82, 84) jeweils einen umfänglich geschlossenen oder teilumfänglich offenen Fingerring (40, 42) zum Durchstecken bzw. Anlegen eines Zeige- und/oder Mittelfingers aufweisen.

6. Handgriff nach einem der Ansprüche 1 bis 5, **dadurch gekennzeichnet, daß** das Daumen-Griffteil (34; 88) in zumindest zwei Arbeitsstellungen, in denen das Daumen-Griffteil (34; 88) mit jeweils einem der beweglichen Griffteile (30, 32; 82, 84) die scherenartig bedienbare Griffanordnung bildet, am Handgriffkörper (28; 86) drehfest arretiert ist.

7. Handgriff nach Anspruch 6, **dadurch gekennzeichnet, daß** das Daumen-Griffteil (34; 88) beim Erreichen der beiden Arbeitsstellungen mit dem Handgriffkörper (28; 86) selbsttätig verrastet.

8. Handgriff nach einem der Ansprüche 1 bis 7, **dadurch gekennzeichnet, daß** das Daumen-Griffteil (34; 88) in zumindest einer Drehrichtung über einen Vollkreis drehbar ist.

9. Handgriff nach einem der Ansprüche 1 bis 8, **dadurch gekennzeichnet, daß** das Daumen-Griffteil (34; 88) am Handgriffkörper (28; 86) relativ zu diesem axial bewegbar angebracht ist, wobei das Daumen-Griffteil (34; 88) in Richtung proximales Ende in eine axial nach proximal vom Handgriffkörper (28; 86) zurückgezogene Verdrehstellung bewegbar ist, in der es verdrehbar ist.

10. Handgriff nach Anspruch 9, **dadurch gekennzeichnet, daß** das Daumen-Griffteil (34; 88) und der Handgriffkörper (28; 86) mittels einer axial trennbaren Stift-Loch-Anordnung drehfest arretiert sind.

11. Handgriff nach Anspruch 9 oder 10, **dadurch gekennzeichnet, daß** das Daumen-Griffteil (34; 88) mittels einer zwischen diesem und dem Handgriffkörper (28; 86) wirkenden Druckfeder (68) nach distal vorgespannt ist.

12. Handgriff nach einem der Ansprüche 1 bis 11, **dadurch gekennzeichnet, daß** die beweglichen Griffteile (30, 32; 82, 84) verschwenkbar am Handgriffkörper (28; 86) angeordnet sind.

13. Handgriff nach einem der Ansprüche 1 bis 12, **dadurch gekennzeichnet, daß** Arretiermittel (92) vorgesehen sind, die in einer Arbeitsstellung des Daumen-Griffteils (88), in der das Daumen-Griffteil (88) mit dem einen beweglichen Griffteil (82) die scherenartig bedienbare Griffanordnung bildet, das mit dem anderen beweglichen Griffteil (84) verbundene Maulteil in seiner Schließlage unbeweglich arretiert, und umgekehrt.

14. Handgriff nach Anspruch 13, **dadurch gekennzeichnet, daß** die beweglichen Griffteile (82, 84) jeweils über eine axial bewegliche Zugstange (96, 100) mit dem zugehörigen Maulteil in Wirkverbindung stehen, und daß die Arretiermittel (92) die jeweilige Zustange (96, 100) in einer Position unbeweglich arretieren, in der das zugehörige Maulteil geschlossen ist.

15. Handgriff nach Anspruch 14, **dadurch gekennzeichnet, daß** die Arretiermittel (92) eine drehbare Hülse (110) aufweisen, die am distalen Ende des Daumen-Griffteils (88) angeordnet ist, die an einem Innenumfangsabschnitt einen nach innen ragenden Vorsprung (112) aufweist, der in eine Ausnehmung (114, 116) in der jeweiligen zu arretierenden Zugstange (96, 100) eingreift, um diese Zugstange (96, 100) zu arretieren.

16. Handgriff nach Anspruch 15, **dadurch gekennzeichnet, daß** der Vorsprung (112) mit der Ausnehmung (114, 116) durch Drehen des Daumen-Griffteils (88) in die gewünschte Arbeitsstellung in Eingriff kommt.

17. Medizinisches Instrument, mit zwei unabhängig voneinander beweglichen Maulteilen (14, 16), **gekennzeichnet durch** einen Handgriff (10; 80) nach einem der Ansprüche 1 bis 16.

18. Medizinisches Instrument nach Anspruch 17, **dadurch gekennzeichnet, daß** die Schließrichtung des jeweiligen Maulteils (14, 16) und die Bewegungsrichtung des zugehörigen beweglichen Griffteils (30, 32; 82, 84) bezüglich des Daumen-Griffteils (34; 88) in etwa der gleichen Ebene liegt.

## Claims

1. A hand grip for a medical instrument (12) which has two jaw parts (14, 16) movable independently of one another, comprising a hand grip body (28; 86) on which two grip elements (30, 32; 82, 84), movable independently of one another, for selectively actuating the jaw parts (14, 16) are arranged, and a thumb grip element (34; 88) which has a thumb ring (38; 90), the movable grip elements (30, 32; 82, 84) being arranged in a manner spaced distally from the thumb grip element (34; 88) on the hand grip body (28; 86), and protruding laterally therefrom, **characterized in that** the thumb grip element (34; 88) is arranged at the proximal end of the hand grip body (28; 86), and the thumb grip element (34; 88) is rotatable about a longitudinal hand grip axis (36; 91) so that the one and the other movable grip element (30, 32; 82, 84) is movable alternately into a position forming in each case with the thumb grip element (34; 88) a hand grip arrangement operable in scissor fashion, in which position the thumb ring (38; 90) and the grip element (30, 32; 82, 84) forming with it the hand grip arrangement operable in scissor fashion lie substantially in a plane.

2. The hand grip of claim 1, **characterized in that** the two movable grip elements (30, 32; 82, 84) are arranged in circumferentially spaced-apart fashion, and form with the thumb ring (38; 90) substantially an isosceles triangle arrangement.

3. The hand grip of claim 1 or 2, **characterized in that** the thumb ring (38; 90) and the two grip elements (30, 32; 82, 84) are together arranged in one plane.

4. The hand grip of anyone of claims 1 through 3, **characterized in that** the thumb ring (90) protrudes laterally from the hand grip axis (91) in such a way that, with the grip element (82, 84) that together with it forms the scissor-like hand grip arrangement, it lies approximately at one level in terms of the spacing from the longitudinal hand grip axis (91).

5. The hand grip of anyone of claims 1 through 4, **characterized in that** the movable grip elements (30, 32; 82, 84) each have a finger ring (40, 42), peripherally closed or open over part of the periphery, for insertion or placement of an index and/or middle finger.

6. The hand grip of anyone of claims 1 through 5, **characterized in that** the thumb grip element (34; 88) is nonrotatably locked on the hand grip body (28; 86) in at least two working positions in which the thumb grip element (34; 88) forms, with one of the movable grip elements (30, 32; 82, 84) in each case, the hand grip arrangement that is actuable in scissor-hand grip fashion.

7. The hand grip of claim 6, **characterized in that** the thumb grip element (34; 88) automatically snap-locks to the grip body (28; 86) when the two rotational positions are reached.

8. The hand grip of anyone of claims 1 through 7, **characterized in that** the thumb grip element (34; 88) is rotatable through a full circle in at least one rotation direction.

9. The hand grip of anyone of claims 1 through 8, **characterized in that** the thumb grip element (34; 88) is mounted on the hand grip body (28; 86) in axially movable fashion relative thereto, the thumb grip element (34; 88) being movable in the direction of the proximal end into a rotational position pulled axially in the proximal direction back from the hand grip body (28; 86), in which it is rotatable.

10. The hand grip of claim 9, **characterized in that** the thumb grip element (34; 88) and the hand grip body (28; 86) are nonrotatably locked by way of an axially separable pin-and-hole connection.

11. The hand grip of claim 9 or 10, **characterized in that** the thumb grip element (34; 88) is pre-loaded in the distal direction by way of a compression spring (68) acting between it and the hand grip body (28; 86).

12. The hand grip of anyone of claims 1 through 11, **characterized in that** the movable grip elements (30, 32; 82, 84) are arranged pivotably on the hand grip body (28; 86).

13. The hand grip of anyone of claims 1 through 12, **characterized in that** locking means (92) are provided which, when the thumb grip element (88) is in a working position in which the thumb grip element (88) forms the hand grip arrangement operable in scissor fashion with the one movable grip element (82), locks the jaw part joined to the other movable grip element (84) in its closed position, and vice versa.

14. The hand grip of claim 13, **characterized in that** the movable grip elements (82, 84) are each in working engagement with the associated jaw part via an axially movable pull rod (96, 100); and the locking means (92) immovably lock the respective pull rod (96, 100) in a position in which the associated jaw part is closed.

15. The hand grip of claim 14, **characterized in that** the locking means (92) have a rotatable sleeve (110) that is arranged at the distal end of the thumb grip element (88) and that has on one inner circumferential segment an inwardly projecting protrusion (112) that, in order to lock the respective pull rod (96, 100) that is to be locked, engages into a recess **in that** pull rod (96, 100).

16. The hand grip of claim 15, **characterized in that** the protrusion (112) comes into engagement with the recess (114, 116) by rotation of the thumb grip element (88) into the desired working position.

17. A medical instrument having two jaw parts (14, 16) movable independently of one another, **characterized by** a hand grip (10; 80) as defined in anyone of claims 1 through 16.

18. The medical instrument of claim 17, **characterized in that** the closing direction of the respective jaw part (14, 16) and the movement direction of the corresponding movable grip element (30, 32; 82, 84) with respect to the thumb grip element (34; 88) lie in approximately the same plane.

## Revendications

1. Poignée pour un instrument médical (12) qui est muni de deux becs (14, 16) mobiles indépendamment l'un de l'autre, comportant un corps de poignée (28 ; 86), sur lequel sont disposés deux éléments de prise (30, 32 ; 82, 84), mobiles indépendamment l'un de l'autre pour actionner au choix les becs (14, 16), et comportant un élément de prise pour le pouce (34 ; 88), qui comporte une bague pour le pouce (38 ; 90), les éléments de prise (30, 32 ; 82, 84) mobiles étant disposés à distance de l'élément de prise pour le pouce (34 ; 88) sur le corps de poignée (28 ; 86) et en s'avançant en saillies latérales sur celui-ci, **caractérisée en ce que** l'élément de prise pour le pouce (34 ; 88) est disposé sur l'extrémité proximale du corps de poignée (28 ; 86) et **en ce que** l'élément de prise pour le pouce (34 ; 88) est rotatif autour d'un axe longitudinal (36 ; 91) de la poignée, de telle sorte que l'un et l'autre éléments de prise (30, 32 ; 82, 84) mobiles se déplacent en alternance dans une position formant respectivement avec l'élément de prise pour le pouce (34 ; 88) un système de prise à manipuler comme des ciseaux, dans laquelle position la bague pour le pouce (38 ; 90) et l'élément de prise (30, 32 ; 82, 84), formant avec celle-ci le système de prise de type ciseaux, sont sensiblement situés dans un plan parallèle à l'axe longitudinal (36 ; 91) de la poignée.

2. Poignée selon la revendication 1, **caractérisée en ce que** les deux éléments de prise (30, 32 ; 82, 84) mobiles sont disposés à distance l'un de l'autre dans le sens du pourtour et forment avec la bague pour le pouce (38 ; 90) un agencement en triangle sensiblement isocèle.

3. Poignée selon la revendication 1 ou 2, **caractérisée en ce que** la bague pour le pouce (38 ; 90) et les deux éléments de prise (30, 32 ; 82, 84) sont disposés conjointement dans un même plan.

4. Poignée selon l'une des revendications 1 à 3, **caractérisée en ce que** la bague pour le pouce (90) s'écarte sur le côté de l'axe (91) de la poignée de telle sorte qu'elle est située, par rapport à l'élément de prise (82, 84), formant avec elle un système de prise de type ciseaux, à pratiquement la même hauteur par rapport à la distance avec l'axe longitudinal (91) de la poignée.

5. Poignée selon l'une des revendications 1 à 4, **caractérisée en ce que** les éléments de prise (30, 32 ; 82, 84) mobiles comportent chacun une bague (40, 42), fermée sur son pourtour ou partiellement ouverte sur son pourtour, pour faire passer ou mettre en appui l'index et/ou le majeur.

6. Poignée selon l'une des revendications 1 à 5, **caractérisée en ce que** l'élément de prise pour le pouce (34 ; 88) peut être immobilisé en rotation sur le corps de poignée (28 ; 86) dans au moins deux positions de travail, dans lesquelles l'élément de prise pour le pouce (34 ; 88) forme avec respectivement un des éléments de prise (30, 32 ; 82, 84) mobiles un système de prise de type ciseaux.

7. Poignée selon la revendication 6, **caractérisée en ce que** l'élément de prise pour le pouce (34 ; 88) se bloque automatiquement sur le corps de poignée (28 ; 86) lorsqu'il parvient dans les deux positions de travail.

8. Poignée selon l'une des revendications 1 à 7, **caractérisée en ce que** l'élément de prise pour le pouce (34 ; 88) est rotatif sur un cercle complet dans au moins un sens de rotation.

9. Poignée selon l'une des revendications 1 à 8, **caractérisée en ce que** l'élément de prise pour le pouce (34 ; 88) est monté sur le corps de poignée (28 ; 86) de manière mobile axialement par rapport à celui-ci, l'élément de prise pour le pouce (34 ; 88) étant rendu mobile en direction de l'extrémité proximale dans une position de rotation extraite du corps de poignée (28 ; 86) axialement vers l'extrémité proximale, dans laquelle position il est rotatif.

10. Poignée selon la revendication 9, **caractérisée en ce que** l'élément de prise pour le pouce (34 ; 88) et le corps de poignée (28 ; 86) peuvent être bloqués en rotation au moyen d'un système à téton et trou, séparable dans le sens axial.

11. Poignée selon la revendication 9 ou 10, **caractérisée en ce que** l'élément de prise pour le pouce (34 ; 88) est précontraint vers l'extrémité distale au moyen d'un ressort de compression (68) agissant entre ledit élément de prise pour le pouce et le corps de poignée (28 ; 86).

12. Poignée selon l'une des revendications 1 à 11, **caractérisée en ce que** les éléments de prise (30, 32 ; 82, 84) mobiles sont montés pivotants sur le corps de poignée (28 ; 86).

13. Poignée selon l'une des revendications 1 à 12, **caractérisée en ce qu'**il est prévu des moyens de blocage (92) qui, dans une position de travail de l'élément de prise pour le pouce (88), dans laquelle l'élément de prise pour le pouce (88) forme avec un élément de prise (82) mobile le système de type ciseaux, bloquent de manière immobile dans une position de fermeture le bec relié à l'autre élément de prise (84) mobile, et inversement.

14. Poignée selon la revendication 13, **caractérisée en ce que** les éléments de prise (82, 84) sont respectivement en liaison active avec le bec correspondant par l'intermédiaire d'une tige de traction (96, 100) mobile, et **en ce que** les moyens de blocage (92) bloquent la tige de traction (96, 100) correspondante de manière immobile dans une position, dans laquelle le bec correspondant est fermé.

15. Poignée selon la revendication 14, **caractérisée en ce que** les moyens de blocage (92) comportent une douille rotative (110), qui est disposée au niveau de l'extrémité distale de l'élément de prise pour le pouce (88), qui comporte sur une partie de son pourtour extérieur une saillie (112) orientée vers l'intérieur, qui s'engage dans un évidement (114, 116) de la tige de traction (96, 100) à bloquer correspondante, en vue de bloquer cette tige de traction (96, 100).

16. Poignée selon la revendication 15, **caractérisée en ce que** la saillie (112) entre en prise avec l'évidement (114, 116) par la rotation de l'élément de prise pour le pouce (88) dans la position de travail souhaitée.

17. Instrument médical comportant deux becs (14, 16) mobiles indépendamment l'un de l'autre, **caractérisé par** une poignée (10 ; 80) selon l'une des revendications 1 à 16.

18. Instrument médical selon la revendication 17, **caractérisé en ce que** la direction de fermeture de chaque bec (14, 16) et la direction de déplacement des éléments de prise (30, 32 ; 82, 84) mobiles correspondants par rapport à l'élément de prise pour le pouce (34 ; 88) sont situées pratiquement dans le même plan.
